# FASCICULE DE BREVET EUROPEEN

(11) **EP 1 433 475 B1**
(45) Date de publication et mention de la délivrance du brevet: **29.11.2006**
(21) Numéro de dépôt: 03293325.1
(22) Date de dépôt: 24.12.2003
(51) Int. Cl.: A61K 8/31, A61K 8/19, A61K 8/34, A61Q 5/10

(54) **Composition colorante contenant une phase mésomorphe et son procédé de préparation, composition prête à l'emploi pour la teinture des matières kératiniques**
Farbmittelzusammensetzung enthaltend eine mesomorphe Phase, Verfahren zur Herstellung und gebrauchsfertige Zusammensetzung zum Färben von keratinischen Fasern
Dyestuff composition comprising a mesomorphic phase, process of its preparation and a ready-to-use composition for dyeing keratinous fibres

(30) Priorité: 24.12.2002 FR 0216599
(43) Date de publication de la demande: 30.06.2004
(73) Titulaire: L'ORÉAL, 75008 Paris (FR)
(72) Inventeur: Nicolas-Morgantini, Luc, 60810 Rully (FR); Petit, Jean-Marc, 92110 Clichy (FR)
(74) Mandataire: Casalonga, Axel

(56) Documents cités:
- EP-A- 0 312 343
- WO-A-02/41983
- WO-A-99/30676
- DE-A- 4 005 008

## Description

La présente invention concerne une composition colorante, un procédé de préparation d'une telle composition, une composition prête à l'emploi pour la teinture d'oxydation des fibres kératiniques, en particulier des cheveux, un procédé de teinture ainsi qu'un kit de coloration.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para-phénylènediamines, des ortho- ou para-aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des méta-phénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Ces compositions de coloration comprennent généralement de l'ammoniac comme agent alcalinisant et lors de leur application sur les cheveux se produit un dégagement d'ammoniac entraînant une odeur désagréable.

La Demanderesse a trouvé de manière surprenante et inattendue que l'on pouvait limiter ce dégagement d'ammoniac de manière importante en ajoutant une phase mésomorphe dans une composition colorante comprenant au moins un colorant d'oxydation et de l'ammoniac, en une quantité d'au moins 10 % en poids par rapport au poids total de ladite composition.

On obtient alors une limitation importante du dégagement d'ammoniac lors de l'application sur les fibres kératiniques, d'une composition prête à l'emploi comprenant ladite composition colorante, que l'ammoniac soit contenu dans la phase mésomorphe ou à l'extérieur de cette phase mésomorphe.

Par fibres kératiniques, on entend au sens de la présente invention les cheveux, les cils et les sourcils.

La présente invention a donc pour objet une composition colorante comprenant, dans un milieu aqueux approprié pour la teinture, au moins un colorant d'oxydation, de l'ammoniac et au moins 10 % de phase mésomorphe par rapport au poids total de la composition colorante.

L'invention concerne également un procédé de préparation de ladite composition.

Un autre objet de l'invention est une composition prête à l'emploi pour la teinture des fibres kératiniques, en particulier des cheveux.

L'invention a encore pour objet une composition oxydante.

Un objet supplémentaire de l'invention est un kit de coloration.

D'autres objets, caractéristiques, aspects et avantages de l'invention apparaîtront encore plus clairement à la lecture de la description et des divers exemples qui suivent.

De préférence, selon l'invention, la composition colorante comprend, dans un milieu aqueux approprié pour la teinture, au moins un colorant d'oxydation, de l'ammoniac, et au moins une phase mésomorphe en une quantité d'au moins 10 % en poids, mieux encore d'au moins 15 % en poids par rapport au poids total de la composition.

De préférence, ladite composition comprend la phase mésomorphe en une quantité inférieure ou égale à 85 % en poids de la composition colorante.

Par phase mésomorphe, on entend un état intermédiaire entre un état cristallin et un état liquide. La phase mésomorphe utilisée dans la composition selon l'invention est de préférence choisie parmi les phases hexagonales inverses (H₂), lamellaires (L_{α} et L_{β}) et cubiques inverses (I₂ et V₂). On utilise de préférence dans l'invention, une phase lamellaire L_{β}.

Par phase hexagonale inverse (H₂), on entend un arrangement hexagonal de micelles cylindriques parallèles de molécules amphiphiles (Handbook of lipid research 4, the physical chemistry of lipids from alkanes to phospholipids, D. M. Small Editor, 1986, Plenum Press, p. 51-56).

Par phase lamellaire fluide ou phase L_{α}, on entend une phase dans laquelle les molécules de tensioactifs et/ou plus généralement de composés amphiphiles s'organisent sous forme de couches bimoléculaires espacées par des feuillets aqueux. Au sein des couches bimoléculaires, les molécules sont réparties selon une géométrie hexagonale ou orthorhombique et leurs chaînes grasses sont dans un état liquide, elles sont orientées perpendiculairement au plan des couches bimoléculaires mais n'ont pas d'orientation spécifique les unes par rapport aux autres dans le plan de ces couches (Handbook of lipid research 4, the physical chemistry of lipids from alkanes to phospholipids, D. M. Small Editor, 1986, Plenum Press, p. 51-56).

Par phase lamellaire gel ou phase L_{β}, on entend une phase dans laquelle les molécules de tensioactifs et/ou plus généralement de composés amphiphiles s'organisent sous forme de couches bimoléculaires espacées par des feuillets aqueux. Au sein des couches bimoléculaires, les molécules sont réparties selon une géométrie hexagonale ou orthorhombique et leurs chaînes hydrocarbonées sont dans un état cristallin, elles sont orientées perpendiculairement au plan des couches bimoléculaires mais n'ont pas d'orientation spécifique les unes par rapport aux autres dans le plan de ces couches (Handbook of lipid research 4, the physical chemistry of lipids from alkanes to phospholipids, D. M. Small Editor, 1986, Plenum Press, p. 51-56).

Par phase cubique, on entend une phase organisée de manière bipolaire en domaines hydrophile et lipophile distincts, en contact étroit et formant un réseau tridimensionnel à symétrie cubique. Une telle organisation a été notamment décrite dans "La Recherche", Vol. 23, pages 306-315, mars 1992 et dans "Lipid Technology", Vol. 2, n° 2, pages 42-45, avril 1990. Selon l'arrangement des domaines hydrophile et lipophile, la phase cubique est dite de type direct ou inverse. La phase cubique inverse correspond à une phase continue huileuse.

La structure de phase mésomorphe peut être vérifiée par microscopie de polarisation et diffusion de rayons X aux petits angles ou toute autre méthode bien connue dans la technique.

Selon la présente invention, la phase mésomorphe comprend de préférence au moins un tensioactif non ionique mono- ou polyoxyalkyléné ou mono- ou polyglycérolé présentant une HLB inférieure ou égale à 10, de préférence comprise entre 1 et 5.

La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256, ou la HLB déterminée par voie expérimentale et telle que décrite dans l'ouvrage des auteurs F. PUISIEUX et M. SEILLER, intitulé "GALENICA 5 : Les systèmes dispersés - Tome I - Agents de surface et émulsions - Chapitre IV - Notions de HLB et de HLB critique, pages 153-194 - paragraphe 1.1.2. Détermination de HLB par voie expérimentale, pages 164-180.

Par tensioactifs non-ioniques mono- ou polyoxyalkylénés, on entend selon l'invention des tensioactifs non-ioniques qui portent dans leur molécule un ou plusieurs groupements oxyalkylène choisis parmi les groupements suivants -CH₂-CH₂-O-, -CH₂-CH₂-CH₂-O-, -CH₂-CH(CH₃)-O- ou leurs mélanges.

Par tensioactifs non-ioniques mono- ou polyglycérolés, on entend selon l'invention des tensioactifs non-ioniques qui portent dans leur molécule un ou plusieurs groupements glycérol.

Des exemples de tensioactifs utilisables dans la présente invention, sont les alcools gras polyglycérolés, les alcools gras mono- ou polyoxyéthylénés, les amides gras mono- ou polyglycérolés comportant en moyenne de 1 à 5 groupements glycérol, les alkylphénols mono- ou polyoxyéthylénés, les condensats d'oxydes d'éthylène et de propylène sur des alcools gras, les huiles végétales polyoxyéthylénées, les esters d'acides gras et de polyéthylèneglycols, les esters d'acides gras et de sorbitol polyoxyéthylénés, et leurs mélanges.

A titres d'exemples de tensioactifs non ioniques présentant une HLB > 5, on peut notamment citer ceux vendus sous les marques commerciales suivantes :

Imbentin POA/024 (HLB=5,5) par la société ICI, Synperonic PE L92 (HLB= 5,5) par la société ICI, Mergital LM2 (HLB=5,8) par la société HENKEL, Atlas G-70140 (HLB=6) par la société ICI, Imbentin. AG/124S/ 020 (HLB=6) par la société KOLB, Imbentin. L/125/025 HLB=6 par la société KOLB, Simulsol 989 (HLB=6) par la société SEPPIC, Soprophor HR10 (HLB=6) par la société RHONE POULENC, Kotilen O/1/050 (HLB=6,2)par la société KOLB, Croduret 10 (HLB=6,3) par la société CRODA, Etocas 10 (HLB=6,3) par la société CRODA, Imbentin OA/030 (HLB=6,3) par la société KOLB, Soprophor 208 (HLB=6,9) par la société RHONE POULENC, Ethylan 172 (HLB=7) par la société HARCROS, Akyporox NP 40 (HLB=7,1) par la société CHEM-Y, Polychol 5 (HLB=7,3) par la société CRODA, Arlatone 985 (HLB=7,5) par la société ICI, Sandoxylate FOL4 (HLB=7,5) par la société SANDOZ, Radiasurf 7453 (HLB=7,8) par la société OLEOFINA, Prox-onic OA-1/04 (HLB=7,9) par la société PROTEX, Prox-onic TD-1/03 (HLB=7,9) par la société PROTEX, Genapol PF 40 (HLB=8) par la société HOECHST, PGE-400 - DS (HLB=8) par la société HEFTI, PGE-400- DO (HLB=8) par la société HEFTI, Sapogenat 6-040 (HLB=8) par la société HOECHST, Intrasol FA28/50/4 (HLB=8,1) par la société STOCKHAUSEN, Serdox NOG 200 S (HLB=8,5) par la société SERVO, Berol 26 (HLB=8,9) par la société BEROL NOBEL, Genapol O-050 (HLB=9) par la société HOECHST, Prox-onic LA-1/04 (HLB=9,2) par la société PROTEX, Eumulgin O5 (HLB=9,5) par la société HENKEL, Etocas 20 (HLB=9,6) par la société CRODA, Antarox CO 520 (HLB=10) par la société RHONE POULENC, Imbentin POA/060 (HLB=10) par la société KOLB, TO-55-EL (HLB=10) par la société HEFTI.

On peut également citer à titres d'exemples de tensioactif non ionique présentant une HLB ≤ 5, ceux vendus sous les marques commerciales suivantes :

Synperonic PE L121 (HLB=0,5) par la société ICI, Prox-Onic EP 4060-1 (HLB=1) par la société PROTEX, Synperonic PE L101 (HLB= 1) par la société ICI, Etocas 29 (HLB=1,7) par la société CRODA, Genapol PF 10 (HLB=2) par la société HOECHST, Synperonic PE L81 (HLB=2) par la société ICI, Prox-Onic EP 1090-1 (HLB=3) par la société PROTEX, Sinnopal DPN2 (HLB=3,3) par la société HENKEL, Antarox CA 210 (HLB=3,5) par la société RHONE-POULENC, Antarox O1P (HLB=3,5) par la société RHONE-POULENC, Alkasurf OP11 (HLB=3,6) par la société RHONE-POULENC, Triton X15 (HLB=3,6) par la société ROHM et HAAS, Alkasurf OP1 (HLB=3,6) par la société RHONE-POULENC, Arlacel 121 (HLB=3,8) par la société ICI, Prox-Onic HR ou HRH-05 (HLB=3,8) par la société PROTEX, Etocas 5 (HLB=3,9) par la société HOECHST, Genapol PF20 (HLB=4) par la société HOECHST, Imbentin N/7 A (HLB=4) par la société KOLB, Synperonic PE L122 (HLB=4) par la société ICI, Ethylan NP1 (HLB=4,5) par la société HARCROS, Imbentin N/020 (HLB=4,5) par la société KOLB, Kotilen O/3/020 (HLB=4,5) par la société KOLB, Synperonic PE L31 (HLB=4,5) par la société ICI, TO-55-A (HLB=4,5) par la société HEFTI, Alkasurf NP-1 (HLB=4,6) par la société RHONE-POULENC, Antarox CO 210 (HLB=4,6) par la société RHONE-POULENC, Prox-Onic NP-1 (HLB=4,6) par la société PROTEX, Rhodiasurf NP2 (HLB=4,6) par la société RHONE-POULENC, Soprophor BC2 (HLB=4,6) par la société RHONE-POULENC, Triton N17 (HLB=4,6) par la société ROHM et HAAS, Akyporox NP15 (HLB=4,7) par la société CHEM-Y, Texofor M2 (HLB=4,8) par la société RHONE-POULENC, Alkasurf SA2 (HLB=4,9) par la société RHONE-POULENC, Arlacel 989 (HLB=4,9) par la société ICI, Brij 72 (HLB=4,9) par la société ICI, Brij 92 (HLB=4,9) par la société ICI, Brij 93 (HLB=4,9) par la société ICI, Prox-Onic SA-1 ou 2/02 (HLB=4,9) par la société PROTEX, Simulsol 72 (HLB=4,9) par la société SEPPIC, Simulsol 92 (HLB=4,9) par la société SEPPIC, Volpo S-2 (HLB=4,9) par la société CRODA, Arlacel 581 (HLB=5,0) par la société ICI, Arlacel 582 (HLB=5,0) par la société ICI, Genapol 0-020 (HLB=5,0) par la société HOECHST, Imbentin POA/020 (HLB=5,0) par la société KOLB, et Mergital Q2 (HLB=5,0) par la société HENKEL.

Un tensioactif non ionique convenant particulièrement bien à l'invention est l'alcool hexadécylique à 2 moles de glycérol.

Le ou les tensioactifs non ioniques sont de préférence présents en une quantité allant de 5 à 30 % en poids, mieux encore de 10 à 20 % en poids par rapport au poids total de la composition colorante.

La phase mésomorphe peut également comprendre en outre au moins un alcool gras comportant de 8 à 30 atomes de carbone, de préférence de 12 à 22 atomes de carbone. A titre d'exemples d'alcools gras, on peut notamment citer l'hexadécanol.

L'alcool gras est de préférence présent en une quantité allant de 3 à 20 % en poids, mieux encore de 5 à 15 % en poids, par rapport au poids total de la composition colorante.

L'ammoniac est de préférence présent dans la composition colorante selon l'invention, en une quantité de 0,1 à 5 % en poids, mieux encore de 1 à 3 % en poids par rapport au poids total de la composition.

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques telles que décrites ci-dessous, ainsi que leurs sels d'addition avec un acide.

Comme para-phénylènediamines, on peut notamment citer celles répondant à la formule (I) suivante, et leurs sels d'addition avec un acide : 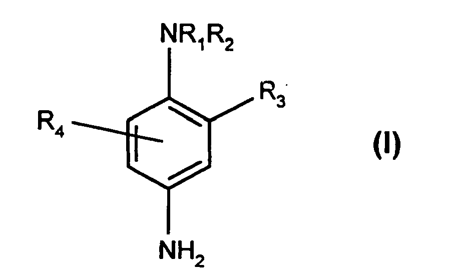 dans laquelle :
R₁ représente un atome d'hydrogène, un groupe alkyle en C₁-C₄, monohydroxyalkyle en C₁-C₄, polyhydroxyalkyle en C₂-C₄ , alcoxy(C₁-C₄)alkyle(C₁-C₄), alkyle en C₁-C₄ substitué par un groupement azoté, phényle ou 4'-aminophényle ;
R₂ représente un atome d'hydrogène, un groupe alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido ;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un groupe alkyle en C₁-C₄.

Parmi les groupements azotés de la formule (I) ci-dessus, on peut notamment citer les groupes amino, monoalkyl(C₁-C₄)amino, dialkyl(C₁-C₄)amino, trialkyl(C₁-C₄)amino, monohydroxyalkyl(C₁-C₄)-amino, imidazolinium et ammonium.

A titre d'exemples de paraphénylènediamines de formule (I) ci-dessus, on peut notamment citer la paraphénylènediamine, la para-toluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino-2-chloro-aniline, la 2-(β-hydroxyéthyl-paraphénylènediamine, la 2-fluoro-paraphénylène-diamine, la 2-isopropyl-paraphénylènediamine, la N-(β-hydroxy-propyl)-paraphénylènediamine, la 2-hydroxyméthyl-paraphénylène-diamine, la N,N-diméthyl-3-méthyl-paraphénylènediamine, la N,N-(éthyl,β-hydroxyéthyl)-paraphénylènediamine, la N-(β,γ-dihydroxy-propyl)-paraphénylènediamine, la N-(4'-aminophényl)-paraphénylène-diamine, la N-phényl-paraphénylènediamine, la 2-β-hydroxyéthyloxy-paraphénylènediamine, la 2-β-acétylaminoéthyloxy-paraphénylène-diamine, la N-(β-méthoxyéthyl)-paraphénylènediamine, la 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la para-toluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions colorantes conformes à l'invention, on peut notamment citer les composés répondant à la formule (II) suivante, et leurs sels d'addition avec un acide : 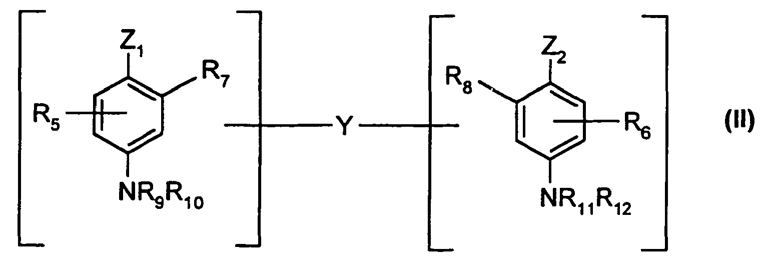 dans laquelle :
- Z₁ et Z₂, identiques ou différents, représentent un groupe hydroxyle ou -NH₂ pouvant être substitué par un groupe alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs groupes hydroxyle ou alcoxy en C₁-C₆ ;
- R₅ et R₆ représentent un atome d'hydrogène ou d'halogène, un groupe alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un groupe alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les groupes amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis(4'-aminophényl)-éthylènediamine, la N,N'-bis(4-amino-phényl)-tétraméthylènediamine, la N,N'-bis-(β-hydroxyéthyl)-N,N'-bis(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diamino-phénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

A titre de para-aminophénol, on peut notamment citer ceux répondant à la formule (III) suivante, et leurs sels d'addition avec un acide : 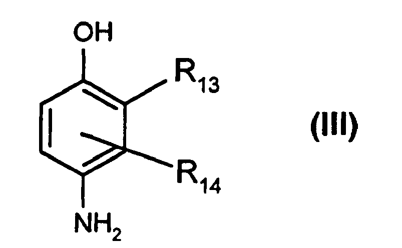 dans laquelle :
R₁₃ représente un atome d'hydrogène, un atome d'halogène tel que le fluor, un groupe alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un groupe alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxy-méthyl-phénol, le 4-amino-2-méthyl-phénol, le 4-amino-2-hydroxy-méthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans les compositions colorantes conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triamino-pyrimidine, la 2,4-dihydroxy-5,6-diaminopyrimidine, la 2,5,6-tri-aminopyrimidine, et les dérivés pyrazolo-pyrimidiniques tels ceux mentionnés dans la demande de brevet FR-A-2 750 048 et parmi lesquels on peut notamment citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol ; le 2-(7-amino-pyrazolo-[1,5-a]-pyrimidin-3-ylamino)-éthanol ; le 2-[(3-amino-pyrazolo-[1,5-a]-pyrimidin-7-yl)-(2-hydroxy-éthyl)-amino]-éthanol ; le 2-[(7-amino-pyrazolo-[1,5-a]-pyrimidin-3-yl)-(2-hydroxy-éthyl)-amino]-éthanol ; la 5,6-diméthyl-pyrazolo-[1,5-a]-pyrimidine-3,7-diamine ; la 2,6-diméthyl-pyrazolo-[1,5-a]-pyrimidine-3,7-diamine ; la 2, 5, N7, N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)-amino-1-méthyl-pyrazole, et leurs sels d'addition avec un acide.

Selon la présente invention, les bases d'oxydation représentent de préférence de 0,0005 à 12% en poids environ, et encore plus préférentiellement de 0,005 à 8% en poids environ du poids total de la composition.

Les coupleurs utilisables dans la composition colorante selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diamino-phénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut également contenir, en plus des colorants d'oxydation définis ci-dessus, des colorants directs pour enrichir les nuances en reflets. Ces colorants directs peuvent être notamment choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition colorante de l'invention peut contenir en outre une quantité efficace d'un ou plusieurs adjuvants bien connus en coloration d'oxydation, comme par exemple, des agents tensioactifs différents de ceux décrits précédemment, bien connus de la technique et de type anionique, cationique, non-ionique, amphotère ou zwittérionique ou leurs mélanges, des agents épaississants, des parfums, des agents séquestrants tels que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, des vitamines ou provitamines comme le panthénol, des opacifiants.

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la tert-butyl-hydroquinone et l'acide homo-gentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-dessus, de telle manière que les propriétés avantageuses liées intrinsèquement à la composition colorante selon l'invention ne soient pas, ou substantiellement pas, altérées par la ou les adjonctions envisagées.

Le milieu aqueux approprié pour la teinture comprend de l'eau et éventuellement des solvants organiques acceptables sur le plan cosmétique, tels que par exemple, des alcools comme l'alcool éthylique, l'alcool isopropylique, l'alcool benzylique, et l'alcool phényléthylique, ou des glycols ou éthers de glycol comme, par exemple, les éthers monométhylique, monoéthylique et monobutylique d'éthylèneglycol, le propylèneglycol ou ses éthers comme, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

Un mode de réalisation particulièrement intéressant de l'invention consiste à utiliser un milieu aqueux ne contenant pas de solvant organique.

La présente invention a également pour objet un procédé de préparation de la composition colorante telle que définie ci-dessus. Il comprend les étapes de :
a) mélange d'au moins un tensioactif non ionique présentant une HLB inférieure ou égale à 10, de préférence comprise entre 1 et 5, avec de l'eau et, éventuellement, avec un ou plusieurs alcools gras, pour préparer la phase mésomorphe, et
b) addition d'au moins un colorant d'oxydation.

L'ammoniac est ajouté dans l'étape a) et/ou dans l'étape b), en d'autres termes, il est soit ajouté au moment de la préparation de la phase mésomorphe, soit déjà présent dans le milieu de la composition colorante avant addition de la phase mésomorphe, soit ajouté dans la composition colorante après incorporation de la phase mésomorphe.

Un autre mode de réalisation consiste à incorporer une phase mésomorphe contenant de l'ammoniac dans un milieu contenant lui-même de l'ammoniac.

Un autre objet de l'invention consiste en une composition prête à l'emploi pour la teinture d'oxydation des fibres kératiniques, en particulier des cheveux. Elle comprend dans un milieu approprié pour la teinture, une composition colorante (A) telle que décrite ci-dessus, et une composition (B) comprenant au moins un agent oxydant.

La phase mésomorphe est de préférence présente dans cette composition prête à l'emploi en une quantité d'au moins 5 % en poids, mieux encore comprise entre 10 et 30 % en poids par rapport au poids total de la composition prête à l'emploi.

Dans la composition oxydante (B), l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

La composition oxydante peut également contenir en outre une phase mésomorphe.

Le pH de la composition colorante (A) ou de la composition de teinture prête à l'emploi et appliquée sur les fibres kératiniques [composition résultant du mélange de la composition tinctoriale (A) et de la composition oxydante (B)], est généralement compris entre les valeurs 4 et 12. Il est de préférence compris entre 6 et 11, et peut être ajusté à la valeur désirée au moyen d'agents acidifiants ou alcalinisants additionnels bien connus de l'état de la technique en teinture des fibres kératiniques.

Parmi les agents alcalinisants additionnels on peut citer, à titre d'exemple, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (IV) suivante : 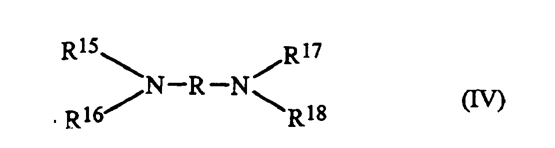 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-dessus, sur les fibres kératiniques sèches ou humides, et à la laisser agir pendant un temps de pause variant, de préférence, 1 à 60 minutes environ, et plus préférentiellement de 10 à 45 minutes environ, à rincer les fibres, puis éventuellement à les laver au shampoing, puis à les rincer à nouveau et à les sécher.

L'invention concerne également un dispositif à plusieurs compartiments ou kit pour la teinture d'oxydation des fibres kératiniques, et en particulier des cheveux, comportant au moins deux compartiments dont l'un d'entre eux renferme une composition colorante (A) telle que définie ci-dessus, et l'autre, une composition oxydante (B) telle que définie ci-dessus.

Les exemples suivants sont donnés à titre illustratif de l'invention.

### EXEMPLES

### Exemple 1 : Préparation d'une phase mésomorphe lamellaire L_{β}

On a ajouté de l'eau et de l'ammoniac à un mélange d'alcool hexadécylique à 2 moles de glycérol (C₁₆G₂) et d'hexadécanol (C₁₆OH), les proportions étant indiquées dans le tableau 1 ci-dessous. On a fait subir à la structure trois cycles thermiques de deux heures, en faisant varier la température de la température ambiante à une température de l'ordre de 70 °C, sous agitation.

On a également préparé une composition aqueuse pour servir de témoin, ainsi qu'une dispersion de la phase lamellaire L_{β} dans de l'eau (ou dispersion L_{β}).

**Tableau 1**

| | % en poids | | | |
|---|---|---|---|---|
| | C₁₆G₂ | C₁₆OH | NH₃ | Eau |
| Composition aqueuse | - | - | 1 | 99 |
| Phase lamellaire L_{β} | 52,5 | 32,5 | 1 | 14 |
| Dispersion L_{β} | 10,5 | 6,5 | 1 | 82 |

On a vérifié la structure de la phase lamellaire par microscopie de polarisation et diffusion des rayons X aux petits angles.

On a ensuite mesuré le dégagement d'ammoniac avec des tubes colorimétriques, sur des échantillons de 10 ml de chacune des compositions, contenus dans des flacons de 30 ml, ouverts. L'incertitude résultante est d'environ 10 % au maximum. Les mesures pour chaque échantillon ont duré de 20 à 30 secondes.

| | Dégagement NH₃ (ppm) au bout de | | | |
|---|---|---|---|---|
| | 1 min. | 5 min. | 10 min. | 20 min. |
| Composition aqueuse | > 1400 | 1367 | 1300 | 1133 |
| Phase lamellaire L_{β} | 433 | 283 | 183 | 133 |
| Dispersion L_{β} | 533 | 333 | 367 | 350 |

L'échantillon de phase lamellaire diffuse de 3 à 8 fois moins que l'échantillon témoin.

La dispersion de phase lamellaire dans un excès d'eau présente un dégagement 3 à 4 fois inférieur à celui de l'échantillon témoin. On retrouve ce résultat que l'ammoniac soit présent dans la phase mésomorphe avant dispersion ou dans l'eau de dispersion.

### Exemple 2 :

On a préparé une composition colorante selon l'invention en mélangeant à température ambiante (de l'ordre de 20 à 25 °C),100 g. de phase lamellaire préparée dans l'exemple 1, et 30 g. de support de coloration dont les composants sont indiqués ci-dessous.

Les composants de la composition colorante, y compris les composants de la phase lamellaire, sont indiqués ci-dessous avec leurs proportions pondérales par rapport au poids total de la composition colorante.

| Composants de la composition colorante : | | % en poids |
|---|---|---|
| - Acide oléique | | 2,1 |
| - 1-méthyl-2,5-diaminobenzène | | 0,31 |
| - Sel pentasodique de l'acide diéthylène-triamine-pentacétique en solution aqueuse à 40% | | 1,5 |
| - Alcool cétylstéarylique | | 12,5 |
| - Thiolactate d'ammonium en solution aqueuse à 58% | | 0,62 |
| - Polycondensat tétraméthyl-hexaméthylènediamine/ 1,3-dichloropropylène en solution aqueuse à 60 % | | 3,9 |
| - Tétrachlorhydrate de 1,3-bis[(4-aminophényl)(2-hydroxyéthyl)amino]-2-propanol | | 0,04 |
| - 1,3-dihydroxybenzène | | 0,001 |
| - 1-hydroxy-4-aminobenzène | | 0,08 |
| - Alcool oléocétylique oxyéthyléné (30 OE) | | 2,8 |
| - Monoéthanolamine | | 0,49 |
| - Alcool oléique | | 2,1 |
| - 1-méthyl-2-hydroxy-4-aminobenzène | | 0,62 |
| - 1-méthyl-2-hydroxy-4-béta-hydroxyéthylamino-benzène | | 0,15 |
| - Ammoniaque (20%) | | 12,0 |
| - Alcool hexadécylique à 2 moles de glycérol | | 12,1 |
| - Hexadécanol | | 7,5 |
| - Eau déionisée | qs | 100 |

On constate une nette diminution de la quantité d'ammoniac dégagé au cours du temps.

## Revendications

1. Composition colorante, **caractérisée en ce qu'**elle comprend, dans un milieu aqueux approprié pour la teinture, au moins un colorant d'oxydation, de l'ammoniac et au moins une phase mésomorphe présente en une quantité d'au moins 10 % en poids par rapport au poids de la composition.

2. Composition colorante selon la revendication 1, **caractérisée en ce que** la quantité de phase mésomorphe est supérieure ou égale 15 % en poids par rapport au poids de la composition.

3. Composition colorante selon la revendication 1 ou 2, **caractérisée en ce que** la quantité de phase mésomorphe est inférieure ou égale à 85 % en poids par rapport au poids total de la composition.

4. Composition colorante selon l'une quelconque des revendications 1 à 3, **caractérisée en ce que** la phase mésomorphe est choisie parmi les phases hexagonales inverses (H₂), lamellaires (L_{α} et L_{β}) et cubiques inverses (I₂ et V₂).

5. Composition colorante selon la revendication 4, **caractérisée en ce que** la phase mésomorphe est une phase lamellaire L_{β}.

6. Composition colorante selon l'une quelconque des revendications 1 à 5, **caractérisée en ce que** la phase mésomorphe comprend au moins un tensioactif non ionique présentant une HLB inférieure ou égale à 10, de préférence comprise entre 1 et 5.

7. Composition colorante selon la revendication 6, **caractérisée en ce que** les tensioactifs non ioniques sont choisis parmi les alcools gras polyglycérolés, les alcools gras mono- ou polyoxyéthylénés, les amides gras mono- ou polyglycérolés comportant en moyenne de 1 à 5 groupements glycérol, les alkylphénols mono- ou polyoxyéthylénés ayant plus de 2 moles d'oxyde d'éthylène, les condensats d'oxydes d'éthylène et de propylène sur des alcools gras, les huiles végétales polyoxyéthylénées ayant plus de 5 moles d'oxyde d'éthylène, les esters d'acides gras et de polyéthylèneglycols, les esters d'acides gras et de sorbitol polyoxyéthylénés, et leurs mélanges.

8. Composition colorante selon la revendication 7, **caractérisée en ce que** le tensioactif non ionique est l'alcool hexadécylique à 2 moles de glycérol.

9. Composition colorante selon l'une quelconque des revendications 6 à 8, **caractérisée en ce que** le tensioactif non ionique est présent en une quantité allant de 5 à 30 % en poids, mieux encore de 10 à 20 % en poids, par rapport au poids total de la composition colorante.

10. Composition colorante selon l'une quelconque des revendications précédentes, **caractérisée en ce que** la phase mésomorphe comprend en outre au moins un alcool gras comportant de 8 à 30 atomes de carbone.

11. Composition colorante selon la revendication 10, **caractérisée en ce que** l'alcool gras est l'hexadécanol.

12. Composition colorante selon la revendication 10 ou 11, **caractérisée en ce que** l'alcool gras est présent en une quantité allant de 3 à 20 % en poids, mieux encore de 5 à 15 % en poids, par rapport au poids total de la composition colorante.

13. Composition colorante selon l'une quelconque des revendications précédentes, **caractérisée en ce qu'**elle comprend l'ammoniac en une quantité de 0,1 à 5 % en poids par rapport au poids total de la composition.

14. Composition colorante selon la revendication 13, **caractérisée en ce qu'**elle comprend l'ammoniac en une quantité de 1 à 3 % en poids par rapport au poids total de la composition.

15. Composition colorante selon l'une quelconque des revendications précédentes, **caractérisée en ce que** le colorant d'oxydation est choisi parmi les bases d'oxydation et/ou les coupleurs.

16. Composition selon l'une quelconque des revendications précédentes, **caractérisée en ce qu'**elle contient au moins une base d'oxydation.

17. Composition selon la revendication 15 ou 16, **caractérisée en ce que** les bases d'oxydation sont choisis parmi les ortho- ou para-phénylènediamines, les bases doubles, les ortho- ou para-aminophénols, et les bases hétérocycliques, ainsi que les sels d'addition de ces composés avec un acide.

18. Composition selon la revendication 17, **caractérisée en ce que** les para-phénylènediamines sont choisies parmi les composés de structure (I) suivante : 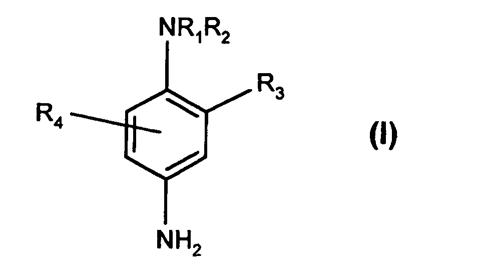 dans laquelle :
R₁ représente un atome d'hydrogène, un groupe alkyle en C₁-C₄, monohydroxyalkyle en C₁-C₄, polyhydroxyalkyle en C₂-C₄ , alcoxy(C₁-C₄)alkyle(C₁-C₄), alkyle en C₁-C₄ substitué par un groupement azoté, phényle ou 4'-aminophényle ;
R₂ représente un atome d'hydrogène, un groupe alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un groupe alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un groupe alkyle en C₁-C₄.

19. Composition selon la revendication 17, **caractérisée en ce que** les bases doubles sont choisies parmi les composés de structure (II) suivante : 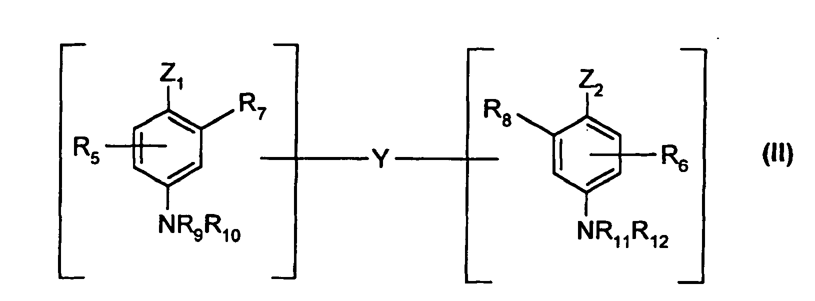 dans laquelle :
- Z₁ et Z₂, identiques ou différents, représentent un groupe hydroxyle ou
- NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs groupes hydroxyle ou alcoxy en C₁-C₆ ;
- R₅ et R₆ représentent un atome d'hydrogène ou d'halogène, un groupe alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un groupe alkyle en C₁-C₄ ; étant entendu que les composés de formule (II) ne comportent qu'un seul bras de liaison Y par molécule.

20. Composition selon la revendication 18 ou 19, **caractérisée en ce que** les groupements azotés sont choisis parmi les groupes amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

21. Composition selon la revendication 17, **caractérisée en ce que** les para-aminophénols sont choisis parmi les composés de structure (III) suivante : 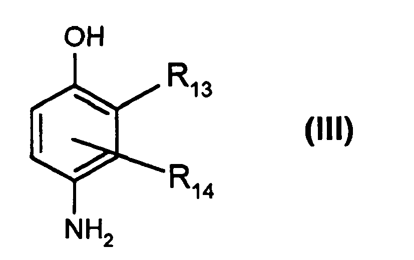 dans laquelle :
R₁₃ représente un atome d'hydrogène,un atome d'halogène tel que le fluor, un groupe alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un groupe alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

22. Composition selon la revendication 17, **caractérisée en ce que** les bases hétérocycliques sont choisies parmi les dérivés pyridiniques, les dérivés pyrimidiniques dont les pyrazolopyrimidines, et les dérivés pyrazoliques.

23. Composition selon l'une quelconque des revendications 15 à 22, **caractérisée en ce que** les bases d'oxydation sont présentes dans des concentrations allant de 0,0005 à 12% en poids par rapport au poids total de la composition.

24. Composition selon la revendication 15, **caractérisée en ce que** les coupleurs sont choisis parmi les métaphénylènediamines, les méta-aminophénols, les métadiphénols, les coupleurs hétérocycliques, et les sels d'addition de ces composés avec un acide.

25. Composition selon la revendication 15 ou 24, **caractérisée en ce que** les coupleurs sont présents dans des concentrations allant de 0,0001 à 10% en poids par rapport au poids total de la composition.

26. Composition selon la revendication 17 ou 24, **caractérisée en ce que** les sels d'addition avec un acide des bases d'oxydation ou des coupleurs sont choisis parmi les chlorhydrates, les bromhydrates, les sulfates, les tartrates, les lactates et les acétates.

27. Composition selon l'une quelconque des revendications précédentes, **caractérisée par le fait qu'**elle contient en outre des colorants directs.

28. Composition selon l'une quelconque des revendications précédentes, **caractérisée en ce qu'**elle comprend en outre un ou plusieurs adjuvants choisis parmi les agents tensioactifs différents de ceux définis dans les revendications 6 et 7, de type anionique, cationique, non-ionique, amphotère ou zwittérionique ou leurs mélanges, des agents épaississants, des parfums, des agents séquestrants, des filtres UV, des cires, des silicones volatiles ou non, cycliques ou linéaires ou ramifiées, organomodifiées ou non, des conservateurs, des céramides, des pseudocéramides, des huiles végétales, minérales ou de synthèse, des vitamines ou provitamines, des opacifiants, et des agents réducteurs ou antioxydants

29. Composition selon l'une quelconque des revendications précédentes, **caractérisée en ce que** le milieu aqueux cosmétiquement acceptable comprend de l'eau et éventuellement des solvants organiques acceptables sur le plan cosmétique.

30. Procédé de préparation de la composition colorante selon l'une quelconque des revendications précédentes, comprenant les étapes de :
a) mélange d'au moins un tensioactif non ionique présentant une HLB inférieure ou égale à 10, de préférence comprise entre 1 et 5, avec de l'eau, et éventuellement avec un ou plusieurs alcools gras, pour préparer la phase mésomorphe, et
b) addition d'au moins un colorant d'oxydation l'ammoniac étant ajouté dans l'étape a) et/ou dans l'étape b).

31. Composition prête à l'emploi, pour la teinture d'oxydation des fibres kératiniques, comprenant, dans un milieu approprié pour la teinture, une composition colorante (A) selon l'une quelconque des revendications 1 à 29, et une composition (B) comprenant au moins au moins un agent oxydant.

32. Composition selon la revendication 31, **caractérisée en ce que** la phase mésomorphe est présente en une quantité d'au moins 5 % en poids par rapport au poids total de la composition.

33. Composition selon la revendication 32, **caractérisée en ce que** la quantité de phase mésomorphe est comprise entre 10 et 30 % en poids par rapport au poids total de la composition.

34. Composition selon l'une quelconque des revendications 31 à 33, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, éventuellement en présence de leur donneur ou cofacteur respectif.

35. Composition selon la revendication 34, **caractérisée en ce que** l'agent oxydant est le peroxyde d'hydrogène.

36. Composition selon la revendication 34, **caractérisée en ce que** l'agent oxydant est une solution d'eau oxygénée dont le titre varie de 1 à 40 volumes.

37. Composition selon l'une quelconque des revendications 31 à 36, **caractérisée en ce qu'**elle présente un pH allant de 4 à 12.

38. Composition oxydante **caractérisée en ce qu'**elle contient au moins un agent oxydant tel que défini dans l'une quelconque des revendications 34 à 36, et une phase mésomorphe telle que définie dans l'une quelconque des revendications 1 à 12.

39. Procédé de teinture, **caractérisé en ce qu'**il consiste à appliquer une composition prête à l'emploi selon l'une quelconque des revendications 31 à 37, réalisée extemporanément au moment de l'emploi sur les fibres kératiniques sèches ou humides, et à la laisser agir pendant un temps de pause variant de 1 à 60 minutes environ, et de préférence de 10 à 45 minutes environ, à rincer les fibres, puis éventuellement à les laver au shampoing, puis à les rincer à nouveau et à les sécher.

40. Dispositif à plusieurs compartiments ou kit pour la teinture d'oxydation des fibres kératiniques, comportant au moins deux compartiments dont l'un d'entre eux renferme une composition colorante (A) selon l'une quelconque des revendications 1 à 29, et l'autre, une composition oxydante (B) telle que définie dans l'une quelconque des revendications 31 et 34 à 36.

## Claims

1. Dye composition comprising, in an aqueous medium that is suitable for dyeing, at least one oxidation dye, ammonia and at least one mesomorphic phase present in an amount of at least 10% by weight relative to the weight of the composition.

2. Dye composition according to Claim 1, **characterized in that** the amount of mesomorphic phase is greater than or equal to 15% by weight relative to the weight of the composition.

3. Dye composition according to Claim 1 or 2, **characterized in that** the amount of mesomorphic phase is less than or equal to 85% by weight relative to the total weight of the composition.

4. Dye composition according to any one of Claims 1 to 3, **characterized in that** the mesomorphic phase is chosen from inverse hexagonal phases (H₂), lamellar phases (L_{α} and L_{β}) and inverse cubic phases (I₂ and V₂).

5. Dye composition according to Claim 4, **characterized in that** the mesomorphic phase is a lamellar phase L_{β}.

6. Dye composition according to any one of Claims 1 to 5, **characterized in that** the mesomorphic phase comprises at least one nonionic surfactant with an HLB value of less than or equal to 10 and preferably between 1 and 5.

7. Dye composition according to Claim 6, **characterized in that** the nonionic surfactants are chosen from polyglycerolated fatty alcohols, monooxyethylenated or polyoxyethylenated fatty alcohols, monoglycerolated or polyglycerolated fatty amides comprising on average from 1 to 5 glycerol groups, monooxyethylenated or polyoxyethylenated alkylphenols containing more than 2 mol of ethylene oxide, condensates of ethylene oxide and of propylene oxide with fatty alcohols, polyoxyethylenated vegetal oils containing more than 5 mol of ethylene oxide, fatty acid esters of polyethylene glycols, polyoxyethylenated fatty acid esters of sorbitol, and mixtures thereof.

8. Dye composition according to Claim 7, **characterized in that** the nonionic surfactant is hexadecyl alcohol containing 2 mol of glycerol.

9. Dye composition according to any one of Claims 6 to 8, **characterized in that** the nonionic surfactant is present in an amount ranging from 5% to 30% by weight, and more preferably from 10% to 20% by weight relative to the total weight of the dye composition.

10. Dye composition according to any one of the preceding claims, **characterized in that** the mesomorphic phase also comprises at least one fatty alcohol containing from 8 to 30 carbon atoms.

11. Dye composition according to Claim 10, **characterized in that** the fatty alcohol is hexadecanol.

12. Dye composition according to Claim 10 or 11, **characterized in that** the fatty alcohol is present in an amount ranging from 3% to 20% by weight and more preferably from 5% to 15% by weight relative to the total weight of the dye composition.

13. Dye composition according to any one of the preceding claims, **characterized in that** it comprises ammonia in an amount of from 0.1% to 5% by weight relative to the total weight of the composition.

14. Dye composition according to Claim 13, **characterized in that** it comprises ammonia in an amount of from 1% to 3% by weight relative to the total weight of the composition.

15. Dye composition according to any one of the preceding claims, **characterized in that** the oxidation dye is chosen from oxidation bases and/or couplers.

16. Composition according to any one of the preceding claims, **characterized in that** it contains at least one oxidation base.

17. Composition according to Claim 15 or 16, **characterized in that** the oxidation bases are chosen from ortho- or para-phenylenediamines, double bases, ortho- or para-aminophenols and heterocyclic bases, and the addition salts of these compounds with an acid.

18. Composition according to Claim 17, **characterized in that** the para-phenylenediamines are chosen from the compounds of structure (I) below: 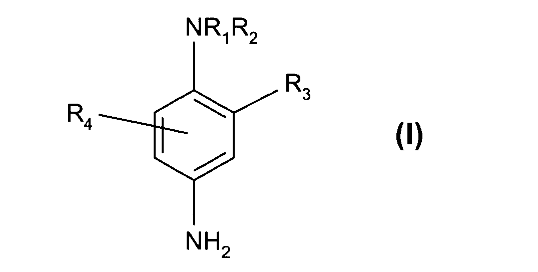 in which:
R₁ represents a hydrogen atom, a C₁-C₄ alkyl group, a C₁-C₄ monohydroxyalkyl group, a C₂-C₄ polyhydroxyalkyl group, a (C₁-C₄)alkoxy(C₁-C₄)alkyl group, a C₁-C₄ alkyl group substituted with a nitrogenous group, a phenyl group or a 4'-aminophenyl group;
R₂ represents a hydrogen atom, a C₁-C₄ alkyl group, a C₁-C₄ monohydroxyalkyl group, a C₂-C₄ polyhydroxyalkyl group, a (C₁-C₄)alkoxy(C₁-C₄)alkyl group or a C₁-C₄ alkyl group substituted with a nitrogenous group;
R₁ and R₂ may also form with the nitrogen atom that bears them a 5- or 6-membered nitrogenous heterocycle optionally substituted with one or more alkyl, hydroxy or ureido groups;
R₃ represents a hydrogen atom, a halogen atom such as a chlorine atom, a C₁-C₄ alkyl group, a sulfo group, a carboxy group, a C₁-C₄ monohydroxyalkyl group, a C₁-C₄ hydroxyalkoxy group, an acetylamino(C₁-C₄)alkoxy group, a mesylamino(C₁-C₄)alkoxy group or a carbamoylamino(C₁-C₄)alkoxy group,
R₄ represents a hydrogen or halogen atom or a C₁-C₄ alkyl group.

19. Composition according to Claim 17, **characterized in that** the double bases are chosen from the compounds of structure (II) below: 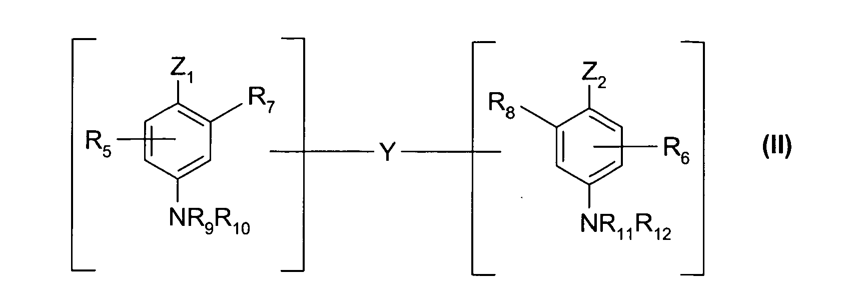 in which
- Z₁ and Z₂, which may be identical or different, represent a hydroxyl or -NH₂ group which may be substituted with a C₁-C₄ alkyl group or with a linking element Y;
- the linking element Y represents a linear or branched alkylene chain containing from 1 to 14 carbon atoms, which may be interrupted by or terminated with one or more nitrogenous groups and/or one or more hetero atoms such as oxygen, sulfur or nitrogen atoms, and optionally substituted with one or more hydroxyl or C₁-C₆ alkoxy groups;
- R₅ and R₆ represent a hydrogen or halogen atom, a C₁-C₄ alkyl group, a C₁-C₄ monohydroxyalkyl group, a C₂-C₄ polyhydroxyalkyl group, a C₁-C₄ aminoalkyl group or a linking element Y;
- R₇, R₈, R₉, R₁₀, R₁₁ and R₁₂, which may be identical or different, represent a hydrogen atom, a linking element Y or a C₁-C₄ alkyl group;
it being understood that the compounds of formula (II) contain only one linking element Y per molecule.

20. Composition according to Claim 18 or 19, **characterized in that** the nitrogenous groups are chosen from amino, mono(C₁-C₄)alkylamino, di(C₁-C₄)alkylamino, tri(C₁-C₄)alkylamino, monohydroxy(C₁-C₄)alkylamino, imidazolinium and ammonium groups.

21. Composition according to Claim 17, **characterized in that** the para-aminophenols are chosen from the compounds of structure (III) below: 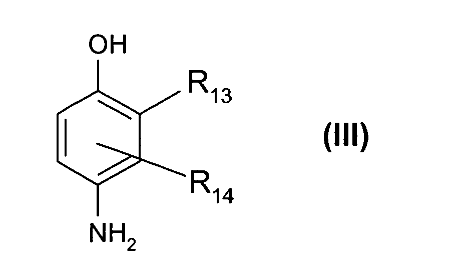 in which:
R₁₃ represents a hydrogen atom, a halogen atom such as fluorine, a C₁-C₄ alkyl, C₁-C₄ monohydroxyalkyl, (C₁-C₄)alkoxy(C₁-C₄)alkyl, C₁-C₄ aminoalkyl or hydroxy(C₁-C₄)alkylamino(C₁-C₄)alkyl group,
R₁₄ represents a hydrogen atom or a halogen atom such as fluorine, a C₁-C₄alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy-(C₁-C₄)alkyl group.

22. Composition according to Claim 17, **characterized in that** the heterocyclic bases are chosen from pyridine derivatives, pyrimidine derivatives including pyrazolopyrimidines, and pyrazole derivatives.

23. Composition according to any one of Claims 15 to 22, **characterized in that** the oxidation bases are present in concentrations ranging from 0.0005% to 12% by weight relative to the total weight of the composition.

24. Composition according to Claim 15, **characterized in that** the couplers are chosen from meta-phenylenediamines, meta-aminophenols, meta-diphenols and heterocyclic couplers, and the addition salts of these compounds with an acid.

25. Composition according to Claim 15 or 24, **characterized in that** the couplers are present in concentrations ranging from 0.0001% to 10% by weight relative to the total weight of the composition.

26. Composition according to Claim 17 or 24, **characterized in that** the addition salts with an acid of the oxidation bases or couplers are chosen form the hydrochlorides, hydrobromides, sulfates, tartrates, lactates and acetates.

27. Composition according to any one of the preceding claims, **characterized in that** it also contains direct dyes.

28. Composition according to any one of the preceding claims, **characterized in that** it also comprises one or more adjuvants chosen from surfactants other than those defined in Claims 6 and 7, of anionic, cationic, nonionic, amphoteric or zwitterionic type or mixtures thereof, thickeners, fragrances, sequestering agents, UV-screening agents, waxes, volatile or non-volatile, cyclic, linear or branched, organomodified or non-organomodified silicones, preserving agents, ceramides, pseudoceramides, vegetal, mineral or synthetic oils, vitamins or provitamins, opacifiers and reducing agents or antioxidants.

29. Composition according to any one of the preceding claims, **characterized in that** the cosmetically acceptable aqueous medium comprises water and optionally cosmetically acceptable organic solvents.

30. Process for preparing the dye composition according to any one of the preceding claims, comprising the steps of:
a) mixing at least one nonionic surfactant with an HLB value of less than or equal to 10, preferably between 1 and 5, with water and optionally with one or more fatty alcohols, to prepare the mesomorphic phase, and
b) adding at least one oxidation dye
the ammonia being added in step a) and/or in step b).

31. Ready-to-use composition for the oxidation dyeing of keratin fibres, comprising, in a medium that is suitable for dyeing, a dye composition (A) according to any one of Claims 1 to 29, and a composition (B) comprising at least one oxidizing agent.

32. Composition according to Claim 31, **characterized in that** the mesomorphic phase is present in an amount of at least 5% by weight relative to the total weight of the composition

33. Composition according to Claim 32, **characterized in that** the amount of mesomorphic phase is between 10% and 30% by weight relative to the total weight of the composition.

34. Composition according to any one of Claims 31 to 33, **characterized in that** the oxidizing agent is chosen from hydrogen peroxide, urea peroxide, alkali metal bromates or ferricyanides, persalts, and redox enzymes such as laccases, peroxidases, and 2-electron oxidoreductases, optionally in the presence of the respective donor or co-factor thereof.

35. Composition according to Claim 34, **characterized in that** the oxidizing agent is hydrogen peroxide.

36. Composition according to Claim 34, **characterized in that** the oxidizing agent is an aqueous hydrogen peroxide solution whose titre ranges from 1 to 40 volumes.

37. Composition according to any one of Claims 31 to 36, **characterized in that** it has a pH ranging from 4 to 12.

38. Oxidizing composition, **characterized in that** it contains at least one oxidizing agent as defined in any one of Claims 34 to 36 and a mesomorphic phase as defined in any one of Claims 1 to 12.

39. Dyeing process, **characterized in that** it consists in applying a ready-to-use composition according to any one of Claims 31 to 37, prepared extemporaneously at the time of use, to the wet or dry keratin fibres, and in leaving it to act for a leave-in time ranging from 1 to 60 minutes approximately and preferably from 10 to 45 minutes approximately, rinsing the fibres and then optionally washing them with shampoo, then rinsing them again and drying them.

40. Multi-compartment device or kit for the oxidation dyeing of keratin fibres, comprising at least two compartments, of which one contains a dye composition (A) according to any one of Claims 1 to 29, and the other an oxidizing composition (B) as defined in any one of Claims 31 and 34 to 36.

## Patentansprüche

1. Farbmittelzusammensetzung, **dadurch gekennzeichnet, dass** sie in einem zum Färben geeigneten, wässrigen Medium mindestens einen Oxidationsfarbstoff, Ammoniak und mindestens eine mesomorphe Phase enthält, die in einem Mengenanteil von mindestens 10 Gew.-%, bezogen auf das Gesamtgewicht der Zusammensetzung, enthalten ist.

2. Farbmittelzusammensetzung nach Anspruch 1, **dadurch gekennzeichnet, dass** der Anteil der mesomorphen Phase 15 Gew.-% beträgt oder darüber liegt, bezogen auf das Gesamtgewicht der Zusammensetzung.

3. Farbmittelzusammensetzung nach Anspruch 1 oder 2, **dadurch gekennzeichnet, dass** der Anteil der mesomorphen Phase höchstens 85 Gew.-%, bezogen auf das Gesamtgewicht der Zusammensetzung, beträgt.

4. Farbmittelzusammensetzung nach einem der Ansprüche 1 bis 3, **dadurch gekennzeichnet, dass** die mesomorphe Phase unter den inversen hexagonalen Phasen (H₂), lamellaren Phasen (L_{α} und L_{β}) und inversen kubischen Phasen (I₂ und V₂) ausgewählt ist.

5. Farbmittelzusammensetzung nach Anspruch 4, **dadurch gekennzeichnet, dass** die mesomorphe Phase eine lamellare Phase L_{β} ist.

6. Farbmittelzusammensetzung nach einem der Ansprüche 1 bis 5, **dadurch gekennzeichnet, dass** die mesomorphe Phase mindestens einen nichtionischen grenzflächenaktiven Stoff enthält, der einen HLB-Wert von höchstens 10 und vorzugsweise 1 bis 5 aufweist.

7. Farbmittelzusammensetzung nach Anspruch 6, **dadurch gekennzeichnet, dass** die nichtionischen grenzflächenaktiven Stoffe unter den mehrfach mit Glycerin veretherten Fettalkoholen, mono- oder polyethoxylierten Fettalkoholen, ein- oder mehrfach mit Glycerin veretherten Fettamiden, die im Mittel 1 bis 5 Glyceringruppen enthalten, mono- oder polyethoxylierten Alkylphenolen, die mehr als 2 mol Ethylenoxid enthalten, Kondensaten von Ethylenoxid und Propylenoxid mit Fettalkoholen, polyethoxylierten pflanzlichen Ölen mit mehr als 5 mol Ethylenoxid, Polyethylenglycolfettsäureestern, polyethoxylierten Sorbitfettsäureestern und deren Gemischen ausgewählt sind.

8. Farbmittelzusammensetzung nach Anspruch 7, **dadurch gekennzeichnet, dass** der nichtionische grenzflächenaktive Stoff der Hexadecylalkohol mit 2 mol Glycerin ist.

9. Farbmittelzusammensetzung nach einem der Ansprüche 6 bis 8, **dadurch gekennzeichnet, dass** der nichtionische grenzflächenaktive Stoff in einer Menge von 5 bis 30 Gew.-% und besser 10 bis 20 Gew.-%, bezogen auf das Gesamtgewicht der Farbmittelzusammensetzung, enthalten ist.

10. Farbmittelzusammensetzung nach einem der vorhergehenden Ansprüche, **dadurch gekennzeichnet, dass** die mesomorphe Phase ferner mindestens einen Fettalkohol mit 8 bis 30 Kohlenstoffatomen enthält.

11. Farbmittelzusammensetzung nach Anspruch 10, **dadurch gekennzeichnet, dass** es sich bei dem Fettalkohol um Hexadecanol handelt.

12. Farbmittelzusammensetzung nach Anspruch 10 oder 11, **dadurch gekennzeichnet, dass** der Fettalkohol in einer Menge von 3 bis 20 Gew.-%, besser 5 bis 15 Gew.-%, bezogen auf das Gesamtgewicht der Farbmittelzusammensetzung, vorliegt.

13. Farbmittelzusammensetzung nach einem der vorhergehenden Ansprüche, **dadurch gekennzeichnet, dass** sie Ammoniak in einer Menge von 0,1 bis 5 Gew.-%, bezogen auf das Gesamtgewicht der Zusammensetzung, enthält.

14. Farbmittelzusammensetzung nach Anspruch 13, **dadurch gekennzeichnet, dass** sie Ammoniak in einer Menge von 1 bis 3 Gew.-%, bezogen auf das Gesamtgewicht der Zusammensetzung, enthält.

15. Farbmittelzusammensetzung nach einem der vorhergehenden Ansprüche, **dadurch gekennzeichnet, dass** der Oxidationsfarbstoff unter den Oxidationsbasen und/oder Kupplern ausgewählt ist.

16. Zusammensetzung nach einem der vorhergehenden Ansprüche, **dadurch gekennzeichnet, dass** sie mindestens eine Oxidationsbase enthält.

17. Zusammensetzung nach Anspruch 15 oder 16, **dadurch gekennzeichnet, dass** die Oxidationsbasen unter den ortho- oder paraPhenylendiaminen, Doppelbasen, ortho- oder para-Aminophenolen, heterocyclischen Basen sowie den Additionssalzen dieser Verbindungen mit einer Säure ausgewählt sind.

18. Zusammensetzung nach Anspruch 17, **dadurch gekennzeichnet, dass** die p-Phenylendiamine unter den Verbindungen der folgenden Struktur (I) ausgewählt sind: 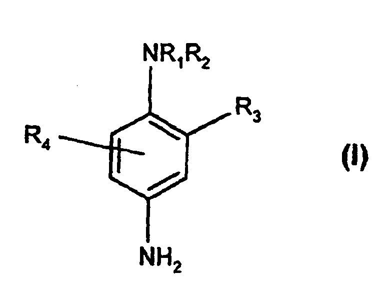 worin bedeuten:
- R₁ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer stickstoffhaltigen Gruppe substituierte C₁₋₄-Alkylgruppe, Phenyl oder 4'-Aminophenyl;
- R₂ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄) oder eine mit einer stickstoffhaltigen Gruppe substituierte C₁₋₄-Alkylgruppe;
wobei die Gruppen R₁ und R₂ gemeinsam mit dem Stickstoffatom, an das sie gebunden sind, einen 5- oder 6-gliedrigen stickstoffhaltigen Heterocyclus bilden können, der gegebenenfalls mit einer oder mehreren Gruppen Alkyl, Hydroxy oder Ureido substituiert ist;
- R₃ ein Wasserstoffatom, ein Halogenatom, beispielsweise ein Chloratom, C₁₋₄-Alkyl, Sulfo, Carboxy, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy;
- R₄ ein Wasserstoffatom, ein Halogenatom oder C₁₋₄-Alkyl.

19. Zusammensetzung nach Anspruch 17, **dadurch gekennzeichnet, dass** die Doppelbasen unter den Verbindungen der folgenden Struktur (II) ausgewählt sind: 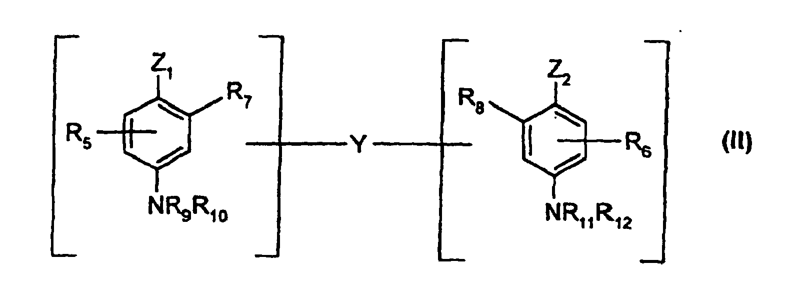 worin bedeuten:
- die Gruppen Z₁ und Z₂, die gleich oder verschieden sind, eine Gruppe Hydroxy oder -NH₂, die mit einer C₁₋₄-Alkylgruppe oder mit einer Verbindungsgruppe Y substituiert sein kann;
- die Verbindungsgruppe Y eine Alkylengruppe mit 1 bis 14 Kohlenstoffatomen, die geradkettig oder verzweigt vorliegt und mit einer oder mehreren stickstoffhaltigen Gruppen und/oder einem oder mehreren Heteroatomen, wie Sauerstoff, Schwefel oder Stickstoff, unterbrochen oder abgeschlossen und gegebenenfalls mit einer oder mehreren Hydroxygruppen oder C₁₋₆-Alkoxygruppen substituiert sein kann;
- R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- die Gruppen R₇, R₈, R₉, Rio, R₁₁ und R₁₂, die gleich oder verschieden sind, ein Wasserstoffatom, eine Verbindungsgruppe Y oder C₁₋₄-Alkyl;
mit der Maßgabe, dass die Verbindungen der Formel (II) nur eine Verbindungsgruppe Y pro Molekül enthalten.

20. Zusammensetzung nach Anspruch 18 oder 19, **dadurch gekennzeichnet, dass** die Stickstoff-haltigen Gruppen unter Amino, Monoalkyl(C₁₋₄)amino, Dialkyl(C₁₋₄)amino, Trialkyl(C₁₋₄)amino, Monohydroxyalkyl(C₁₋₄)amino, Imidazolinium und Ammonium ausgewählt sind.

21. Zusammensetzung nach Anspruch 17, **dadurch gekennzeichnet, dass** die p-Aminophenole unter den Verbindungen der folgenden Struktur (III) ausgewählt sind: 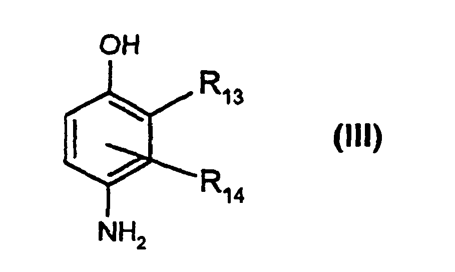 worin bedeuten:
R₁₃ ein Wasserstoffatom, ein Halogenatom, wie Fluor, C₁₋₄-Alkyl, C₁₋₄-Monohydroxyalkyl, Alkoxy(C₁₋₄)alkyl(C₁₋₄), C₁₋₄-Aminoalkyl oder Hydroxy(C₁₋₄)aminoalkyl(C₁₋₄):
R₁₄ ein Wasserstoffatom, ein Halogenatom, wie Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder Alkoxy(C₁₋₄)alkyl(C₁₋₄).

22. Zusammensetzung nach Anspruch 17, **dadurch gekennzeichnet, dass** die heterocyclischen Basen unter den Pyridinderivaten, Pyrimidinderivaten, darunter Pyrazolopyrimidinen, oder Pyrazolderivaten ausgewählt sind.

23. Zusammensetzung nach einem der Ansprüche 15 bis 22, **dadurch gekennzeichnet, dass** die Oxidationsbasen in Konzentrationen von 0,0005 bis 12 Gew.-%, bezogen auf das Gesamtgewicht der Zusammensetzung, enthalten sind.

24. Zusammensetzung nach Anspruch 15, **dadurch gekennzeichnet, dass** die Kuppler unter den m-Phenylendiaminen, m-Aminophenolen, m-Dihydroxybenzolen, heterocyclischen Kupplern und den Additionssalzen dieser Verbindungen mit einer Säure ausgewählt sind.

25. Zusammensetzung nach Anspruch 15 oder 24, **dadurch gekennzeichnet, dass** die Kuppler in Konzentrationen von 0,0001 bis 10 Gew.-%, bezogen auf das Gesamtgewicht der Zusammensetzung, enthalten sind.

26. Zusammensetzung nach einem der Ansprüche 17 bis 24, **dadurch gekennzeichnet, dass** die Additionssalze der Oxidationsbasen oder Kuppler mit einer Säure unter den Hydrochloriden, Hydrobromiden, Sulfaten, Tartraten, Lactaten und Acetaten ausgewählt sind.

27. Zusammensetzung nach einem der vorhergehenden Ansprüche, **dadurch gekennzeichnet, dass** sie ferner Direktfarbstoffe enthält.

28. Zusammensetzung nach einem der vorhergehenden Ansprüche, **dadurch gekennzeichnet, dass** sie ferner einen oder mehrere Zusatzstoff enthält, die unter den grenzflächenaktiven Stoffen vom anionischen, kationischen, nichtionischen, amphoteren oder zwitterionischen Typ, die von den in Anspruch 6 und 7 definierten Tensiden verschieden sind, oder deren Gemischen, Verdickungsmittel, Parfums, Maskierungsmitteln, UV-Filtern, Wachsen, flüchtigen oder nichtflüchtigen, cyclischen, linearen oder verzweigten Siliconen, die gegebenenfalls organomodifiziert sind, Konservierungsmitteln, Ceramiden, Pseudoceramiden, pflanzlichen Ölen, Mineralölen oder synthetischen Ölen, Vitaminen oder Provitaminen, Trübungsmitteln und Reduktionsmitteln und Antioxidantien ausgewählt sind.

29. Zusammensetzung nach einem der vorhergehenden Ansprüche, **dadurch gekennzeichnet, dass** das kosmetisch akzeptable, wässrige Medium Wasser und gegebenenfalls kosmetisch akzeptable, organische Lösungsmittel enthält.

30. Verfahren zur Herstellung der Farbmittelzusammensetzung nach einem der vorhergehenden Ansprüche, **dadurch gekennzeichnet, dass** es die folgenden Schritte umfasst:
a) Mischen mindestens eines nichtionischen grenzflächenaktiven Stoffes, der einen HLB-Wert von 10 oder darunter und vorzugsweise im Bereich von 1 bis 5 aufweist, mit Wasser und gegebenenfalls mit einem oder mehreren Fettalkoholen zur Herstellung der mesomorphen Phase und
b) Zugabe mindestens eines Oxidationsfarbstoffes,
wobei das Ammoniak in Schritt a) und/oder Schritt b) zugegeben wird.

31. Gebrauchsfertige Zusammensetzung zum oxidativen Färben von Keratinfasern, die in einem zum Färben geeigneten Medium eine Farbmittelzusammensetzung (A) nach einem der Ansprüche 1 bis 29 und eine Zusammensetzung (B) enthält, die mindestens ein Oxidationsmittel enthält.

32. Zusammensetzung nach Anspruch 31, **dadurch gekennzeichnet, dass** die mesomorphe Phase in einem Anteil von mindestens 5 Gew.-%, bezogen auf das Gesamtgewicht der Zusammensetzung, enthalten ist.

33. Zusammensetzung nach Anspruch 32, **dadurch gekennzeichnet, dass** die mesomorphe Phase in einer Menge im Bereich von 10 bis 30 Gew.-%, bezogen auf das Gesamtgewicht der Zusammensetzung, enthalten ist.

34. Zusammensetzung nach einem der Ansprüche 31 bis 33, **dadurch gekennzeichnet, dass** das Oxidationsmittel ausgewählt ist unter Wasserstoffperoxid, Harnstoffperoxid, Alkalimetallbromaten, Alkalimetallferricyaniden, Salzen von Persäuren und Redoxenzymen, wie Laccsen, Peroxidasen und Oxidoreduktasen (2 Elektronen), gegebenenfalls mit ihrem jeweiligen Donor oder Cofaktor.

35. Zusammensetzung nach Anspruch 34, **dadurch gekennzeichnet, dass** es sich bei dem Oxidationsmittel um Wasserstoffperoxid handelt.

36. Zusammensetzung nach Anspruch 34, **dadurch gekennzeichnet, dass** das Oxidationsmittel eine Wasserstoffperoxidlösung mit einem Titer von 1 bis 40 Volumina ist.

37. Zusammensetzung nach einem der Ansprüche 31 bis 36, **dadurch gekennzeichnet, dass** sie einen pH-Wert von 4 bis 12 aufweist.

38. Oxidierende Zusammensetzung, **dadurch gekennzeichnet, dass** sie mindestens ein Oxidationsmittel, wie es in einem der Ansprüche 34 bis 36 definiert wurde, und eine mesomorphe Phase, wie sie in einem der Ansprüche 1 bis 12 definiert wurde, enthält.

39. Verfahren zum Färben, **dadurch gekennzeichnet, dass** es darin besteht, auf die trockenen oder feuchten Keratinfasern eine gebrauchsfertige Zusammensetzung nach einem der Ansprüche 31 bis 37 aufzutragen, die bedarfsgemäß bei der Anwendung hergestellt wird, während einer Zeitspanne von etwa 1 bis 60 Minuten und vorzugsweise etwa 10 bis 45 Minuten einwirken zu lassen, die Fasern zu spülen, dann gegebenenfalls mit Haarwaschmittel zu waschen, anschließend nochmals zu spülen und zu trocknen.

40. Vorrichtung mit mehreren Abteilungen oder "Kit" zum oxidativen Färben von Keratinfasern, die mindestens zwei Abteilungen aufweist, wobei eine Abteilung eine Farbmittelzusammensetzung (A) nach einem der Ansprüche 1 bis 29 und die andere Abteilung eine oxidierende Zusammensetzung (B) enthält, wie sie in den Ansprüchen 31 und 34 bis 36 definiert ist.
